# EUROPÄISCHE PATENTSCHRIFT

(11) **EP 1 070 701 B1**
(45) Veröffentlichungstag und Bekanntmachung des Hinweises auf die Patenterteilung: **22.09.2004**
(21) Anmeldenummer: 00111141.8
(22) Anmeldetag: 24.05.2000
(51) Int. Cl.: C07C 69/38, C07C 67/30

(54) **Verfahren zur Herstellung von Alkalimetallsalzen der Malonsäuremonoalkylester**
Process for the preparation of alkali metal salts of the monoalkylester of malonic acid
Procédé de préparation de sels alcalins d'esters monoalkyliques de l'acide malonique

(30) Priorität: 21.07.1999 DE 19934165
(43) Veröffentlichungstag der Anmeldung: 24.01.2001
(73) Patentinhaber: Degussa AG, 40474 Düsseldorf (DE)
(72) Erfinder: Muhr, Jürgen, Dr., 53347 Alfter (DE)

(56) Entgegenhaltungen:
- DE-A- 19 817 101
- NL-A- 7 202 987
- US-A- 3 855 229

## Beschreibung

Die Erfindung betrifft ein Verfahren zur Herstellung von Alkalimetallsalzen der Malonsäuremonoalkylester durch selektive (oder partielle) Verseifung von Malonsäuredialkylestern über die Zwischenstufe eines CH-aciden Alkalimetallsalzes.

Alkalimetallsalze der Malonsäuremonoalkylester und insbesondere das Kaliummonoethylmalonat (im folgenden kurz KEM genannt) werden als Vorprodukte für die Synthese von Pharmawirkstoffen mit Chinolonstruktur verwendet. Der Einsatz für Synthesen im Pharmabereich stellt hohe Ansprüche an die Reinheit der Produkte.

Eine schon im vergangenen Jahrhundert von van't Hoff (Ber.Dtsch.Chem. Ges., 7, 1572) beschriebene und auch heute noch praktizierte Synthese für Kaliummonoethylmalonat geht von Diethylmalonat (im folgenden kurz DEM genannt) aus, das mit Kaliumhydroxid selektiv verseift wird. Gemäß EP 0 720 981 A1 erfolgt dies unter Einsatz äquimolarer Eduktmengen in alkoholischem Medium, d.h. mit alkoholischer Kalilauge. Die angestrebte selektive Verseifung wird allerdings nur in unzureichendem Maße erreicht, so daß man lediglich ein mit Dikaliummalonat (im folgenden kurz DKM genannt) erheblich verunreinigtes Zielprodukt erhält. Der Gehalt an DKM liegt üblicherweise in der Größenordnung von mehreren Gew.-%. Die Abtrennung des DKM aus dem KEM, die für die Verwendung des letzteren als Pharmavorprodukt erforderlich ist, ist schwierig und schließt umfangreiche Reinigungsoperationen ein.

Nachteilig bei dem genannten Verfahren ist auch, daß alkoholische Kalilauge kein handelsübliches Produkt ist, sondern aus Alkohol und festem Kaliumhydroxid (Ätzkali) unter Abführung der Lösungswärme hergestellt werden muß. Die Handhabung und Lagerung von ätzenden Feststoffen, wie Ätzkali und Ätznatron, in technischen Mengen ist aus Sicherheits- und Arbeitsschutzgründen mit erheblichen Kosten verbunden. Zudem können alkoholische Alkalilaugen, insbesondere solche mit Alkoholen mit einer C-Zahl in der Kette von >1, in der Regel nicht gelagert werden, ohne daß Alterungsprozesse einsetzen, die leicht zu einer Produktverfärbung führen.

Ein weiterer Nachteil des bekannten Verfahrens besteht in der relativ starken Verdünnung, in der es durchgeführt werden muß. Der Alkohol wird in der 19 bis 28-fachen Gewichtsmenge, bezogen auf Kaliumhydroxid, eingesetzt. Diese große Alkoholmenge wirkt sich nachteilig auf die Raum-Zeit-Ausbeute aus und erhöht den Aufwand für die Rückgewinnung des Lösemittels. Die Alkoholmenge wird weiterhin dadurch erhöht, daß auch das DEM in alkoholischer Lösung eingesetzt wird.

Box et al., Heterocycles, Vol. 32, No. 2, 1991, 245 ff., erwähnen eine Synthese von β-Lactonen und β-Lactamen, bei der KEM als Zwischenprodukt dient. Im experimentellen Teil wird auf Seite 247 die Herstellung von KEM aus DEM und alkoholischer Kalilauge beschrieben. Die beiden Edukte werden, wie bei dem Verfahren der erwähnten EP 0 720 981 A1, in äquimolaren Mengen eingesetzt. nämlich jeweils 100 mmol. Die molaren Verhältnisse von DEM zu KOH wurden jedoch falsch berechnet, denn 20,225 g DEM sind 126 mmol und entsprechen der 1,26fachen molaren Menge, bezogen auf KOH. Wenn man weiterhin annimmt, daß das eingesetzte Kaliumhydroxid, wie handelsüblich, einen KOH-Gehalt von ca. 90 Gew.-% (Rest Wasser) hatte, entsprechen 20,225 g DEM sogar der 1,38-fachen molaren Menge, bezogen auf KOH (gerechnet 100%). Eine Nacharbeitung mit molaren Verhältnissen von 1,26:1 und von 1,38:1 ergab, daß nach diesem Verfahren zwar ein reines KEM mit weniger als 0,5 Gew.-% DKM erhältlich ist, das ausgefallene KEM jedoch sehr schlecht filtrierbar war. Bei Laboransätzen betrug die Filtrierzeit mehr als zwei Stunden. Solche Zeiten sind für eine Produktion im technischen Maßstab prohibitiv. Zudem werden nach Box et al., ähnlich wie bei dem Verfahren der erwähnten EP 0720 981 A1, erhebliche Mengen an Alkohol benötigt, und schließlich sind auch die Ausbeuten an KEM nicht befriedigend.

Gegenstand der deutschen Patentanmeldung 19817101.3 (O.Z. 5297) ist ein Verfahren zur Herstellung von KEM durch selektive Verseifung von DEM mit Kaliumhydroxid, bei dem man das Kaliumhydroxid dem DEM zufügt, DEM und Kaliumhydroxid in einem molaren Verhältnis von mindestens 1,5 anwendet und das Kaliumhydroxid in dem DEM wirkungsvoll verteilt.

Eine der Aufgaben der vorliegenden Erfindung ist die Bereitstellung eines Verfahrens zur Herstellung von Alkalimetallsalzen der Malonsäuremonoalkylester, das die genannten Nachteile des vorveröffentlichten Standes der Technik vermeidet. d.h. ohne Mitverwendung von großen Mengen an Alkohol und ohne Verwendung von alkoholischer Alkalilauge hohe Ausbeuten an reinen, DKM-armen und leicht filtrierbaren Alkalimetallsalzen der Malonsäuremonoalkylester ergibt. Eine weitere Aufgabe der Erfindung besteht in der Bereitstellung eines Verfahrens, das ähnlich vorteilhaft ist wie das Verfahren der genannten älteren deutschen Patentanmeldung.

Diese Aufgaben wurden erfindungsgemäß gelöst durch ein Verfahren zur Herstellung von Alkalimetallsalzen der Malonsäuremonoalkylester durch selektive Verseifung von Malonsäuredialkylestern mit einer basischen Alkalimetallverbindung, bei dem man in einer ersten Stufe aus dem Malonsäuredialkylester und einem Alkalimetallalkoholat, welche im Molverhältnis von 1 : 1 bis 10 : 1 verwendet werden, ein CH-acides Alkalimetallsalz des Malonsäuredialkylesters herstellt und dieses in einer zweiten Stufe durch Einwirkung von Wasser hydrolysiert.

Das erfindungsgemäße Verfahren läßt sich durch das folgende Formelschema wiedergeben:

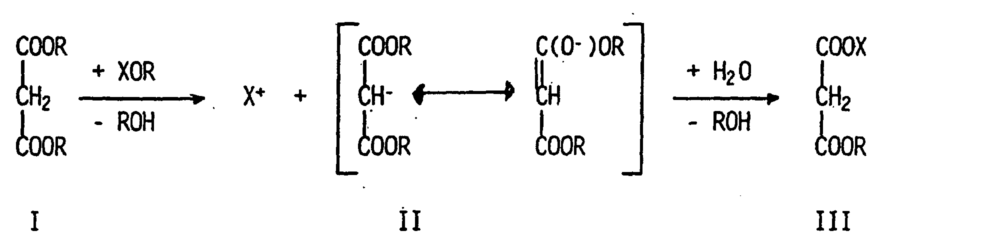

Darin bedeutet X ein Alkalimetall und R einen Alkylrest, vorteilhaft mit 1 bis 4 Kohlenstoffatomen. Vorzugsweise hat R in dem Malonsäuredialkylester I dieselbe Bedeutung wie in dem Alkalimetallalkoholat. weil dann Umesterungen vermieden werden. Das Reaktionsprodukt II der ersten Stufe, in der dem Kation ^{X+} ein mesomeres Anion gegenübersteht, wird für die Zwecke dieser Anmeldung als CH-acides Alkalimetallsalz des Malonesters bezeichnet.

Das erfindungsgemäße Verfahren ergibt Alkalimetallsalze der Malonsäuremonoalkylester III mit einem Gehalt an Dialkalimetallsalzen der Malonsäure, wie DKM, von <1 Gew.-%, und entspricht damit den Anforderungen für Pharmasynthesen. Das Verfahren hat akzeptable Filtrierzeiten und erfordert einen vergleichsweise geringen Energieaufwand zur Rückgewinnung des Alkohols und von überschüssigem Dialkylmalonat. Im Gegensatz zu dem Verfahren der erwähnten älteren deutschen Patentanmeldung läßt sich nicht nur KEM, sondern lassen sich erfindungsgemäß auch andere Alkalimetallsalze des Malonsäuremonoethylesters und lassen sich auch die Alkalimetallsalze anderer Malonsäuremonoalkylester in hoher Reinheit und gut filtrierbarer Form herstellen. Das Verfahren erfordert keine gesonderte Herstellung von alkoholischer Alkalilauge aus den Alkalimetallydroxiden und Alkohol, sondern verwendet die in Form ihrer alkoholischen Lösungen in technischen Mengen verfügbaren und weitgehend alterungsbeständigen Alkalimetallalkoholate.

Ein wesentliches Merkmal des erfindungsgemäßen Verfahrens besteht darin, daß man in einer ersten Stufe aus einem Malonsäuredialkylester I und einem Alkalimetallalkoholat ein CH-acides Alkalimetallsalz des Malonsäuredialkylesters III herstellt. Bevorzugte Malonsäuredialkylester I leiten sich von Alkanolen mit 1 bis 4 Kohlenstoffatomen ab. Besonders bevorzugt wird der Diethylester.

Bevorzugte Alkalimetallalkoholate sind die Natrium- und insbesondere die Kaliumalkoholate. Die Alkalimetallalkoholate werden vorzugsweise in Form der 10- bis 30-gewichtsprozentigen alkoholischen Lösungen eingesetzt, wie sie durch Umsetzung der in technischen Mengen verfügbaren Alkalimetallamalgame mit überschüssigem Alkohol hergestellt werden. Prinzipiell ist es auch möglich, feste Alkalimetallalkoholate einzusetzen. Dann geht jedoch der Vorteil verloren, den Lösungen hinsichtlich der Handhabung und der Dosierung bieten.

Die genannten Edukte können in äquimolaren Mengen eingesetzt werden. vorteilhaft wendet man jedoch den Malonsäuredialkylester I in bis zu 10-fachem molaren Überschuß an. Bevorzugt wird ein 0,5- bis 10-facher, insbesondere ein 2- bis 4-facher molarer Überschuß. Der überschüssige Malonsäuredialkylester wirkt als inertes Löse- oder Verdünnungsmittel. Dem Malonsäuredialkylester und/oder der alkoholischen Alkalimetallalkoholatlösung können weiterhin andere inerte Löse- oder Verdünnungsmittel zugesetzt werden. Als solche eignen sich z.B. aromatische oder nicht-aromatische, gegebenenfalls inert substituierte Kohlenwasserstoffe, wie Toluol, die isomeren Xylole und Ethylbenzol.

Man kann das erfindungsgemäße Verfahren z.B. in einem Rührreaktor oder in einem Strömungsrohrreaktor durchführen. Dabei spielt es keine Rolle, in welcher Reihenfolge man die Edukte miteinander mischt. Man kann also beispielsweise den Malonsäuredialkylester in die alkoholische Lösung des Alkalialkoholats einbringen, die alkoholische Alkalimetallalkoholatlösung in den gegebenenfalls in einem inerten Löseoder Verdünnungsmittel gelösten Malonsäuredialkylester einleiten oder beide Edukte gleichzeitig in einen Strömungsreaktor oder in einen Rührreaktor einbringen, in dem ein Teil des Malonsäuredialkylesters und/oder ein inertes Löse- oder Verdünnungsmittel vorgelegt war.

Man führt die erste Stufe des erfindungsgemäßen Verfahrens zweckmäßig unter Atmosphärendruck und bei einer Temperatur von <80°C, vorzugsweise von <60°C und insbesondere bei 0 bis 30°C durch, vorteilhaft unter Abführung der Reaktionswärme. Die Geschwindigkeit, mit der das CH-acide Alkalimetallsalz II entsteht, hängt u.a. von der Reaktionstemperatur und der Alkoholkomponente im Malonsäuredialkylester I und im Alkalimetallalkoholat ab. Im allgemeinen läuft die Reaktion spontan ab. Das CH-acide Salz scheidet sich als farbloser Feststoff ab. der z.B. durch Filtration aus dem Reaktionsgemisch abgetrennt und in einem inerten Löse- oder Verdünnungsmittel zum Zielprodukt hydrolysiert werden kann. Vorteilhaft verzichtet man jedoch auf eine Abtrennung und führt die Hydrolyse gleich im Reaktionsgemisch der ersten Stufe durch, gegebenenfalls - zur Verbesserung der Raum-Zeit-Ausbeute - nach Entfernung des gesamten oder eines Teiles des überschüssigem Malonsäuredialkylesters und/oder inerten Löse- oder Verdünnungsmittels.

Zur Herstellung des Alkalimetallsalzes des Malonsäuremonoalkylesters III hydrolysiert man in der zweiten Stufe das CH-acide Alkalimetallsalz II mit Wasser, vorteilhaft mit 1 bis 5 Mol und insbesondere mit etwa 1 Mol Wasser je Mol des eingesetzten Alkalimetallalkoholats. Die Hydrolyse erfolgt zweckmäßig unter Atmosphärendruck und bei einer Temperatur von <100°C, vorzugsweise von <60°C und insbesondere bei 0 bis 40°C . Die Hydrolyse erfolgt relativ schnell und ist im allgemeinen in weniger als 5 Stunden abgeschlossen. Längere Hydrolysezeiten sind möglich, aber nicht mit einem besonderen Vorteil verbunden,

Das farblose Zielprodukt III fällt in fester Form an und kann in üblicher Weise aus dem Hydrolysegemisch abgetrennt werden. Es ist problemlos filtrierbar und läßt sich durch Auswaschen. z.B. mit dem Alkohol des korrespondierenden Esters, weiter reinigen. Die Waschflüssigkeit, die Mutterlauge sowie die gegebenenfalls nach der ersten Stufe durch Destillation abgetrennten Anteile des Reaktionsgemisches können nach Ergänzung des verbrauchten Malonsäuredialkylesters I als Edukt und Reaktionsmedium für einen neuen Ansatz verwendet werden. Zweckmäßig trennt man jedoch zumindest einen Teil des Alkohols ab, um einer unerwünschten Verdünnung des Reaktionsmediums entgegenzuwirken. Dies geschieht vorteilhaft unter vermindertem Druck und/oder unter Mitverwendung eines Schleppmittels, das mit dem jeweiligen Alkohol ein Azeotrop bildet. Geeignete Schleppmittel sind z.B. aliphatische und/oder aromatische Kohlenwasserstoffe, wie Cyclohexan.

Die folgenden Beispiele sollen die Erfindung weiter erläutern, nicht aber ihren Anwendungsbereich begrenzen.

### Beispiel 1

Zu 358,4 g (1 mol) einer 23,5 gew.-%igen Lösung von Kaliumethylat in Ethanol fügt man innerhalb 1 h bei 25°C unter Rühren 480,5 g (3 mol) Diethylmalonat (DEM). Danach dosiert man innerhalb von 1 h 18,0 g (1 mol) Wasser hinzu. Der ausgefallene Feststoff wird abfiltriert und zweimal mit jeweils 100 ml Ethanol gewaschen und im Wasserstrahlpumpenvakuum getrocknet. Die Ausbeute an KEM beträgt 142,5 g (83,7 %). dessen Reinheit >99%, bestimmt durch Ionenchromatographie.

### Beispiel 2

Man verfährt wie in Beispiel 1, legt jedoch das Diethylmalonat vor und dosiert die Kaliumethylat-Lösung hinzu. Die Ausbeute an KEM beträgt 85 % d.Th., die Reinheit wiederum > 99%.

### Beispiel 3

Man verfährt wie in Beispiel 1, engt jedoch das Filtrat durch Abdestillieren der Leichtsieder im schwachen Vakuum ein und gibt es dem nächsten, im übrigen gleichen Ansatz zu. Die Ausbeute bei dem nächsten Ansatz beträgt dann >90 % d.Th., die Reinheit wiederum > 90%. Die ses Ergebnis wird weiterhin erzielt, wenn man das eingeengte Filtrat eines Ansatzes dem jeweils nächsten Ansatz zufügt.

## Patentansprüche

1. Verfahren zur Herstellung von Alkalimetallsalzen der Malonsäuremonoalkylester durch selektive Verseifung von Malonsäuredialkylestern mit einer basischen Alkalimetallverbindung, **dadurch gekennzeichnet, daß** man in einer ersten Stufe aus dem Malonsäuredialkylester und einem Alkalimetallalkoholat, welche im Molverhältnis von 1:1 bis 10:1 verwendet werden, ein CH-acides Alkalimetallsalz des Malonsäuredialkylesters herstellt und dieses in einer zweiten Stufe durch Einwirkung von Wasser hydrolysiert.

2. Verfahren nach Anspruch 1, **dadurch gekennzeichnet, daß** die erste Stufe bei einer Temperatur von <80°C durchgeführt wird.

3. Verfähren nach Anspruch 1, **dadurch gekennzeichnet, daß** die erste Stufe bei einer Temperatur von <60°C durchgeführt wird.

4. Verfahren nach Anspruch 1, **dadurch gekennzeichnet, daß** die erste Stufe bei einer Temperatur von 0 bis 30°C durchgeführt wird.

5. Verfahren nach einem der Ansprüche 1 bis 4, **dadurch gekennzeichnet, daß** die Hydrolyse in der zweiten Stufe bei einer Temperatur von <100°C durchgeführt wird.

6. Verfahren nach einem der Ansprüche 1 bis 4, **dadurch gekennzeichnet, daß** die Hydrolyse in der zweiten Stufe bei einer Temperatur von <60°C durchgeführt wird.

7. Verfahren nach einem der Ansprüche 1 bis 4, **dadurch gekennzeichnet, daß** die Hydrolyse in der zweiten Stufe bei einer Temperatur von 0 bis 40°C durchgeführt wird.

8. Verfahren nach einem der Ansprüche 1 bis 7, **dadurch gekennzeichnet, dass** die Hydrolyse in der zweiten Stufe mit 1 bis 5 Mol Wasser je Mol des eingesetzten Alkalimetallalkoholats durchgeführt wird.

9. Verfahren nach einem der Ansprüche 1 bis 8 , **dadurch gekennzeichnet, daß** der Malonsäuredialkylester und das Alkalimetallalkoholat im Molverhältnis von 1,5:1 bis 10:1 verwendet werden.

10. Verfahren nach einem der Ansprüche 1 bis 8 , **dadurch gekennzeichnet, daß** der Malonsäuredialkylester und das Alkalimetallalkoholat im Molverhältnis von 2:1 bis 4:1 verwendet werden.

11. Verfahren nach einem der Ansprüche 1 bis 10, **dadurch gekennzeichnet, daß** die nach Abtrennung des festen Alkalimetallsalzes des Malonsäuremonoaklylesters in der zweiten Stufe erhaltene Mutterlauge sowie etwaige Waschflüssigkeit, gegebenenfalls nach Abtrennung von Alkohol und zusätzlich in die Reaktion eingebrachter inerter, niedrigsiedender Lösemittel und gegebenenfalls zusammen mit nach der ersten Stufe abgetrennten flüchtigen Bestandteilen des Reaktionsgemisches, nach Ergänzung des verbrauchten Malonsäuredialkylesters als Edukt und Reaktionsmedium in das Verfahren zurückgeführt werden.

## Claims

1. A process for preparing an alkali metal salt of a malonic acid monoalkyl ester by selective saponification of a malonic acid dialkyl ester using a basic alkali metal compound, **characterized in that** in a first stage a CH-acid alkali metal salt of the malonic acid dialkyl ester is prepared from the malonic acid dialkyl ester and an alkali metal alkoxide, which are used in a molar ratio of from 1:1 to 10:1, and this is hydrolysed in a second stage by the action of water.

2. A process according to claim 1, **characterized in that** the first stage is carried out at a temperature of <80°C.

3. A process according to claim 1, **characterized in that** the first stage is carried out at a temperature of <60°C.

4. A process according to claim 1, **characterized in that** the first stage is carried out at a temperature of from 0 to 30°C.

5. A process according to any one of claims 1 to 4, **characterized in that** the hydrolysis is carried out in the second stage at a temperature of <100°C.

6. A process according to any one of claims 1 to 4, **characterized in that** the hydrolysis in the second stage is carried out at a temperature of <60°C.

7. A process according to any one of claims 1 to 4, **characterized in that** the hydrolysis in the second stage is carried out at a temperature from 0 to 40°C.

8. A process according to any one of claims 1 to 7, **characterized in that** the hydrolysis in the second stage is carried out with from 1 to 5 mol of water per mol of the alkali metal alkoxide used.

9. A process according to any one of claims 1 to 8, **characterized in that** the malonic acid dialkyl ester and the alkali metal alkoxide are used in a molar ratio of from 1.5:1 to 10:1.

10. A process according to any one of claims 1 to 8, **characterized in that** the malonic acid dialkyl ester and the alkali metal alkoxide are used in a molar ratio of from 2:1 to 4:1.

11. A process according to any one of claims 1 to 10, **characterized in that** the mother liquor obtained after separating off the solid alkali metal salt of the malonic acid monoalkyl ester in the second stage and also any wash liquid, if desired after separating off alcohol and inert low-boiling solvent additionally introduced into the reaction and if desired together with volatile reaction mixture constituents separated off downstream of the first stage, is recirculated to the process as starting material and reaction medium after supplementation of the malonic acid dialkyl ester consumed.

## Revendications

1. Procédé de préparation de sels de métaux alcalins d'ester monoalkyliques de l'acide malonique par saponification sélective d'esters dialkyliques de l'acide malonique à l'aide d'un composé basique de métal alcalin,
**caractérisé en ce que**
dans une première étape, à partir de l'ester dialkylique et d'un alcoolate de métal alcalin utilisés dans un rapport molaire de 1 : 1 à 10 : 1, on prépare un sel CH-acide de métal alcalin de l'ester dialkylique de l'acide malonique, et dans une deuxième étape on l'hydrolyse en faisant agir de l'eau.

2. Procédé selon la revendication 1,
**caractérisé en ce que**
la première étape s'effectue à une température < 80°C.

3. Procédé selon la revendication 1,
**caractérisé en ce que**
la première étape s'effectue à une température < 60°C.

4. Procédé selon la revendication 1,
**caractérisé en ce que**
la première étape s'effectue à une température de 0 à 30°C.

5. Procédé selon l'une des revendications 1 à 4,
**caractérisé en ce que**
dans la seconde étape l'hydrolyse s'effectue à une température < à 100°C.

6. Procédé selon l'une des revendications 1 à 4,
**caractérisé en ce que**
dans la seconde étape l'hydrolyse s'effectue à une température < à 60°C.

7. Procédé selon l'une des revendications 1 à 4,
**caractérisé en ce que**
dans la seconde étape l'hydrolyse s'effectue à une température de O à 40°C.

8. Procédé selon l'une des revendications 1 à 7,
**caractérisé en ce que**
dans la seconde étape l'hydrolyse s'effectue à l'aide de 1 à 5 moles d'eau par mole de l'alcoolate de métal alcalin utilisé.

9. Procédé selon l'une des revendications 1 à 8,
**caractérisé en ce que**
l'ester dialkylique de l'acide malonique et l'alcoolate de métal alcalin sont utilisés dans un rapport molaire de 1,5 : 1 à 10 : 1.

10. Procédé selon l'une des revendications 1 à 8,
**caractérisé en ce que**
l'ester dialkylique de l'acide malonique et l'alcoolate de métal alcalin sont utilisés dans un rapport molaire de 2 : 1 à 4 : 1.

11. Procédé selon l'une des revendications 1 à 10,
**caractérisé en ce que**
le cas échéant après séparation d'alcool et de solvants inertes à bas point d'ébullition introduits additionnellement dans la réaction, et le cas échéant de concert avec des composants volatils du mélange de réaction, séparés après la première étape, l'eau mère obtenue dans la seconde étape après séparation du sel solide de métal alcalin de l'ester dialkylique de l'acide malonique, ainsi qu'un éventuel liquide de lavage, sont ramenés dans le processus en tant qu'éduit et milieu de réaction après avoir remplacé la quantité consommée d'ester dialkylique de l'acide malonique.
